(19) Europäisches Patentamt

European Patent Office

Office européen des brevets

(11) Publication number: **0 267 658 B1**

# EUROPEAN PATENT SPECIFICATION

(45) Date of publication of patent specification: **17.02.93**    (51) Int. Cl.5: **C07C 51/41**, C07C 65/05, C10M 129/54

(21) Application number: **87202185.2**

(22) Date of filing: **09.11.87**

(54) **Process for the preparation of a basic salt, salt thus prepared and lubricating oil compositions containing such a salt.**

(30) Priority: **13.11.86 GB 8627130**

(43) Date of publication of application:
**18.05.88 Bulletin 88/20**

(45) Publication of the grant of the patent:
**17.02.93 Bulletin 93/07**

(84) Designated Contracting States:
**AT BE CH DE ES FR GB GR IT LI LU NL SE**

(56) References cited:
**EP-A- 0 248 465**
**FR-A- 2 074 003**
**GB-A- 780 058**
**GB-A- 786 167**
**GB-A- 1 210 773**

(73) Proprietor: **SHELL INTERNATIONALE RE-SEARCH MAATSCHAPPIJ B.V.**
**Carel van Bylandtlaan 30**
**NL-2596 HR Den Haag(NL)**

(72) Inventor: **de Lind van Wijngaarden, Gerhard**
**Badhuisweg 3**
**NL-1031 CM Amsterdam(NL)**
Inventor: **Kremers, Antoon Paul Michael**
**Badhuisweg 3**
**NL-1031 CM Amsterdam(NL)**
Inventor: **Lammeree, John**
**Badhuisweg 3**
**NL-1031 CM Amsterdam(NL)**

EP 0 267 658 B1

## Description

The present invention relates to a process for the preparation of a basic alkaline earth metal salt of an organic carboxylic acid, a salt thus prepared and to lubricating oil compositions containing such a salt.

The use of alkaline earth metal salts of organic carboxylic acids as additives for lubricating oil compositions is known. The said salts have a dispersant property so that, when applied in such composition, they ensure that the inside of engine cylinders remains clean and that deposition of carbonaceous products on pistons and in piston grooves is counteracted, so that piston-ring sticking is prevented.

It is also known to prepare basic (or overbased) alkaline earth metal salts of such acids. The overbasing provides an alkaline reserve which, when applied in lubricating oil compositions, reacts with and neutralises acidic compounds formed during the operation of the engine in which the composition is applied. Hence, sludge which may arise, is maintained dispersed due to the dispersant property of the salt while acids which would enhance sludge formation are neutralised.

In British patent specification No. 786,167, a process for the preparation of basic salts is described in which an organic acid is reacted with an excess of an alkaline earth metal oxide or hydroxide in a hydrocarbon solvent in the presence of an alcohol and subsequently carbon dioxide is passed through the reaction mixture to yield basic salts. The temperature of the reaction is from about 20°C to 70°C.

In the technical field there is a desire to use products with a basicity as high as possible, i.e. the relative proportion of the organic acid residue in the basic salt is as low as possible. The reason for this is that the costs of the product are mainly incurred by the costs of the organic acid.

The basicity of these products is generally expressed as a basicity index (BI), being defined as the equivalents ratio of the total of alkaline earth metal to the total of organic acid. The prior art processes normally yield products having a BI of at most 10, and it was found that in the preparation of products having a BI that high or even higher a pronounced tendency to gelation occurs, thereby severely hindering the handleability of the products. The present invention provides a process which can yield products which have a very high BI and which show virtually no gelling tendency.

Accordingly, the present invention relates to a process for the preparation of a basic alkaline earth metal salt of an organic carboxylic acid, which comprises:-

(a) preparing a mixture of one equivalent of the organic carboxylic acid and at least 1 equivalent of an alkaline earth metal hydroxide and/or oxide in a hydrocarbon solvent, and optionally a promotor, suitably a $C_{1-6}$ alcohol and/or water;

(b) introducing carbon dioxide into the mixture in an amount of at least 0.5 equivalent carbon dioxide per equivalent alkaline earth metal; and

(c) removing residual solids, if any, and the water layer, if any, obtained; characterised in that the carbonation reaction of step (b) is carried out at a temperature in the range from 75 to 150°C.

Suitable organic carboxylic acids which can be used in the present process, include acids containing a benzene or naphthalene ring and an oil-solubilising radical or radicals having a total of at least 8, in particular at least 12 carbon atoms. Particularly preferred are alkyl salicylic acids having at least 10 carbon atoms in the alkyl group, in particular from 12 to 26 carbon atoms.

Other suitable acids include substituted or unsubstituted aliphatic or cycloaliphatic acids. Examples of these acids include naphthenic acids, and aliphatic acids having more than 8 carbon atoms, such as stearic, isostearic, palmitic, myristic, oleic and hydroxystearic acid, in particular tertiary carboxylic acids, e.g. those sold under the trade name 'VERSATIC' acids (ex Shell Nederland Chemie).

The alkaline earth metal salts prepared include magnesium, calcium, strontium and barium salts. Preferably, the alkaline earth metal applied is magnesium or calcium.

The reaction mixture prepared in step (a) of the present process suitably contains a promotor, preferably an oxygen-containing organic solvent and optionally water. Suitable solvents include $C_{1-6}$ alcohols, polyhydric alcohols such as glycol, propylene glycol, glycerol or 1,3-dihydroxypropane, ethers such as $C_{1-4}$ monoethers of glycol or propylene glycol, di isopropyl ether, 1,3- or 1,4-dioxane, or 1,3-dioxolane. Preferably the promotor is $C_{1-6}$ alcohol, in particular methanol. It will be appreciated that in industrial processes use may be made of technical solvents, and that the use of technically pure promotors, such as methanol, might incur the presence of water. Hence, in such cases addition of water per se is not required since its addition is made implicitly by the addition of the promoter.

The preparation of the mixture according to step (a) of the present process can be carried out at any possible way, e.g. by mixing the alkaline earth metal hydroxide and/or oxide with the alcohol and adding the acid, whether or not in the presence of the alcohol or the hydrocarbon solvent to the resulting mixture. It is preferred to mix the acid and the alkaline earth metal hydroxide and/or oxide in a hydrocarbon solvent and subsequently add the alcohol. The alcohol may contain a substantial amount of water. Preferably the water content is adjusted such that the percentage of water in the mixture amounts to 0 to 10%w, based on the total liquids.

The hydrocarbon solvent can be selected from a wide variety of solvents. Suitable solvents include hydrocarbon oils, such as solvent-refined and/or hydrogenated lubricating oils having a kinematic viscosity of 3.5-35 $mm^2$/s at 100°C. Preferably it is an aromatic hydrocarbon or a hydrocarbon fraction rich in aromatics, such as gasoline. Suitable hydrocarbon solvents are benzene, toluene, xylene or mixtures thereof, xylene being particularly preferred. The alcohol used is preferably methanol. The amount of the solvent is not critical. Alcohol:solvent volume ratios up to 1 can suitably be applied, preference being given to ratios in the range from 0.1 to 0.6.

The concentration of the organic carboxylic acid in the solvent or solvent mixture can vary within wide limits. Suitably the acid equivalent concentration is from 0.01 to 1 molar equivalent/kilogram, preferably from 0.1 to 0.8, based on the combined weight of organic carboxylic acid and hydrocarbon solvent.

The amount of alkaline earth metal to be added in step a) should be at least 1 equivalent, and is preferably more so that the subsequent carbon dioxide supply results in very high BI compounds. In that case, the amount of alkaline earth metal hydroxide and/or oxide added in step a) is preferably from 10 to 25 equivalents per equivalent acid.

The temperature at which step a) is carried out is not critical and may be ambient temperature or elevated temperature. Suitable temperatures include 15-150°C.

In step b) the temperature should be from 75 to 150°C, preferably from 80 to 130°C. In order to obtain the elevated temperature it may be necessary to employ elevated pressures, since the desired reaction temperature may be above the atmospheric reflux temperature of the reaction mixture. Suitable pressures include between 1 and 15 bar abs. Higher pressures are possible, but merely add to the costs of the process. The rate at which the carbon dioxide is introduced is advantageously from 0.05 to 1.0 equivalent carbon dioxide per equivalent acid per minute. The carbon dioxide introduction is conveniently carried out by passing carbon dioxide, or a mixture of carbon dioxide with an inert gas, such as air or nitrogen, through the reaction mixture under slightly higher pressure than the pressure prevailing in the reaction mixture. Higher pressures may be employed. Carbon dioxide will be absorbed in the reaction mixture and will react with the alkaline earth metal compounds present therein forming a basic complex salt of the organic acid salt and carbonate, hydroxide and/or oxide. The amount of carbon dioxide to be taken up in step b) is to a certain extent dependent on the amount of alkaline earth metal added in step a) of the present process. Suitably the relative amount of carbon dioxide is somewhat less than the relative amount of alkaline earth metal hydroxide or oxide.

Preferably the introduction of carbon dioxide in step b) is stopped after 0.5 to 0.9 equivalent carbon dioxide per equivalent alkaline earth metal has been taken up. Conveniently, this corresponds with 5 to 23 equivalent carbon dioxide per equivalent acid.

It has been found that an ageing period between step b) and step c) can be advantageous, since it increases the BI of the resulting basic salt. Such an ageing period amounts suitably to at least 15 min. A maximum period is generally imposed by practical and/or economical reasons, and is generally below 20 hours. Preferably the period between steps b) and c) is from 1 to 4 hrs.

The reaction mixture at the end of step b) may be worked up by any method known in the art. It may be subjected to a centrifuging treatment to remove solids comprising unreacted alkaline earth metal hydroxide and/or oxide and/or non-colloidal alkaline earth metal carbonate, if any. The resulting solution may then be subjected to a liquid-phase separation. One liquid phase is a water layer which may contain the $C_{1-6}$ alcohol when it is used, the other one is the hydrocarbon solvent plus the basic salts dispersed therein. It is also possible to reverse the above operations.

The present process can be used for the preparation of basic salts having a wide variety of basicity indices. So, it would be possible to prepare basic salts having a relatively low BI e.g. from 1 to 10. The present process, however, is excellently suitable for preparing basic salts having a basicity index from 10 to 20.

The process described is a one-step process. However it is possible to integrate the process according to the present invention in a two-step process, in particular in a two-step process according to British patent application No. 8613815 (Applicants' reference K 9731). Thereto, step a) and b) are carried out in two stages, a1, a2, b1 and b2 respectively, whereby the stages comprise:

(a1) preparing a mixture of one equivalent of the organic carboxylic acid and at least one equivalent of an alkaline earth metal hydroxide and/or oxide in a hydrocarbon solvent and optionally a $C_{1-6}$ alcohol and/or water;

(b1) introducing carbon dioxide into the mixture at a temperature from 75 to 150°C until at least 0.5 equivalent carbon dioxide per equivalent alkaline earth metal has been taken up;

(a2) adding at least one further equivalent of alkaline earth metal hydroxide and/or oxide to the reaction mixture, so that the total amount of alkaline earth metal hydroxide and/or oxide is at least 10 equivalent;

(b2) resuming the introduction of carbon dioxide to the resulting mixture at a temperature from 75 to 150°C.

Between stages (b1) and (a2) and after (b2) ageing periods can be employed as is indicated in the above patent application. The ageing period after stage (b2) corresponds with the above-mentioned ageing period between steps (b) and (c).

The process according to the present invention can be carried out batchwise and also in a continuous manner.

The basic salts are excellent dispersant additives in lubricating oils. Therefore the present invention also provides lubricating oil compositions comprising a major amount of a lubricating base oil and a minor amount of a basic alkaline earth metal salt as described hereinbefore.

The lubricating base oil will conveniently constitute more than 50%w of the composition. It can be selected from mineral lubricating oils of varying viscosities, but it also includes a synthetic lubricant, such as ester-type lubricant or a polyolefin-type fluid, or a vegetable oil, or a grease.

Fuel compositions which are used in marine diesel engines usually contain some sulphur compounds. To neutralize the acidic compounds formed from these sulphur compounds a relatively high concentration of the basic salt is employed. Preferably, these marine lubricating oil compositions contain from 5 to 30%w of basic alkaline earth metal salt. Lubricating oil compositions for road engines may contain lower concentrations. The amount of basic alkaline earth metal salt in these lubricating oil compositions is preferably from 0.01 to 5%w, in particular from 0.1 to 4.0%w.

Fuels, such as gasoline, kerosine, diesel fuel and gas oils, can also contain the above basic salts. The amount of these salts is similar to that in road engine lubricating oil compositions or lower; conveniently the amount is from 0.001 to 5%w, in particular from 0.01 to 1.0%w.

The lubricating oil composition can be prepared by mixing a concentrate containing up to 60%w of a basic salt as described above in a lubricating oil, with a lubricating base oil to give the desired concentration. Such a concentrate is conveniently prepared by addition of a lubricating oil to the product obtained after completion of step c), and removal of any volatile hydrocarbon solvent, water and alcohol, if present. The lubricating oil may be the same as the one indicated above as a suitable hydrocarbon solvent. The concentrate may conveniently contain a stabiliser, which is selected from a variety of organic compounds, such as those described in British patent specification No. 818,315. These compounds include mono-or polyhydric alcohols, alkyl amines and alkyl phenols.

The lubricating oil compositions may further contain a number of other additives, such as antioxidants, foam inhibitors, corrosion inhibitors, viscosity index improvers, and pour point depressants, as can be established by a person skilled in the art. In particular, improved properties can be realised by the addition of polyisobutene/succinic anhydride adducts, such as LUBAD 349 (sold by Lubrizol).

The invention will be illustrated by means of the following Examples 5-10; Examples 1-4 being comparative examples..

EXAMPLES 1-5

A number of experiments were carried out in which $C_{14-18}$ alkyl salicylic acid (SA) was taken up in xylene, to which calcium hydroxide was added in such an amount that 17 equivalents of calcium hydroxide were present per equivalent acid. To this mixture methanol, containing 3%w of water, was added in a volume ratio to xylene of about 1:4. The mixture was heated to the reaction temperature while stirring and carbon dioxide was introduced. After a certain uptake the carbon dioxide introduction was stopped. Wherever possible, the reaction mixture was subjected to an ageing period of 19h at a temperature of 55°C. Thereafter the reaction mixture was allowed to settle, yielding two liquid phases, and the water-methanol layer was removed. From the xylene layer residual solids were removed by centrifugation. The basicity index of the salt in the xylene layer was determined by acidimetric titration.

The reaction conditions and the results of the reactions are indicated in Table 1 below.

Table 1

| Example No. | SA concentration, eq/kg | CO$_2$ rate, eq CO$_2$/eq SA/min | CO$_2$ uptake, eq CO$_2$/eq SA | reaction temp. °C | BI eq Ca/eq SA | Comments |
|---|---|---|---|---|---|---|
| 1 | 0.4 | 0.18 | 8.2 | 55 | - | severe gel |
| 2 | 0.4 | 0.08 | 7.8 | 55 | - | severe gel |
| 3 | 0.25 | 0.14 | 9.4 | 55 | - | severe gel |
| 4 | 0.4 | 0.08 | 12.3 | 70 | - | gel at end |
| 5 | 0.4 | 0.17 | 12.3 | 80 | 13.6 | reaction in autoclave at 2 bar, no gel |

From the comparative experiments 1-3 it is apparent that the prior art process, applied to high calcium hydroxide/SA ratios, incurs severe gel formation during the reaction; the reactions had to be stopped after a CO$_2$ uptake of 8.2, 7.8 and 9.4, respectively. Experiment 4 could be continued until the desired CO$_2$ uptake, but gelling occurred at the end of the reaction, rendering it impossible to switch the solvent from xylene to a lubricating oil.

Experiment 5 carried out under the conditions of the present invention yielded a product with a high BI at a suitable viscosity.

150g of the product of Experiment 5 was introduced into 65g of a mineral lubricating oil, the mixture was subjected to vacuum distillation to remove xylene, to yield a 10.9%m/m concentrate, based on calcium content. The kinematic viscosity of this concentrate at 100°C was 32mm__/s.

EXAMPLES 6-10

A further series of experiments was carried out in which $C_{14-18}$ alkylsalicylic acid (SA) was reacted in xylene and methanol having 3%w water, with calcium hydroxide as in Examples 1-5, but using a calcium hydroxide: SA equivalent ratio of 19. The SA concentration in all experiments was 0.4 eq SA/kg xylene and the $CO_2$ introduction was 0.12 eq $CO_2$/eq SA/min. In all experiments an ageing period of 19h (while stirring at 55°C) was used after the $CO_2$ introduction. After the reactions the product was taken up in a lubricating oil concentrate similar to the procedure of Example 5.

In this series the temperature, the pressure and the total amount of $CO_2$ used were varied. The values for these parameters and the results of the Examples are shown in Table 2.

TABLE 2

| Example No. | Reaction conditions | | $CO_2$ uptake | Analysis of oil concentrate | | |
|---|---|---|---|---|---|---|
| | temp. °C | pressure bar | eq $CO_2$/eq SA | BI eq Ca/eq SA | Ca content %w | $V_k$ (100°C) $mm^2/s$ |
| 6 | 85 | 2.5 | 12.0 | 14.0 | 10.0 | 13 |
| 7 | 85 | 2.5 | 13.5 | 15.3 | 9.9 | - |
| 8 | 100 | 3.6 | 12.0 | 14.1 | 9.8 | 11 |
| 9 | 120 | 5.5 | 12.0 | 14.5 | 10.1 | 10 |
| 10 | 120 | 5.6 | 13.5 | 16.2 | 10.2 | 11 |

The products obtained in Examples 6-10 did not show any gelation tendency. Moreover, from comparison of Examples 6, 8 and 9 on the one hand and Examples 7 and 10 on the other it is apparent that higher temperatures at the same $CO_2$ uptake result in products with higher BI.

EXAMPLE 11

This Example illustrates the process according to the present invention carried out in a continuous manner. First the conditions of experiment of Example 8 were repeated, namely with a temperature at the $CO_2$ uptake of 100°C and at a pressure of 3.5 bar. Subsequently, while maintaining the same temperature, pressure and rate of $CO_2$ introduction, a mixture of calcium hydroxide, SA, xylene and methanol (with 3%w water) in the same relative amounts as used in Example 8 was continuously fed into the reaction mixture and at the same time reaction product was withdrawn from the mixture such that the overall Ca/SA/$CO_2$ composition in the reaction mixture remained the same.

Samples were drawn from the reaction product periodically, and taken up in an oil concentrate as in Examples 5-10. Samples 1-3 were taken up in the lubricating oil without applying an ageing period. Sample 4, drawn at the same time as sample 3, was subjected to an ageing period of 19 hrs. at 55°C under stirring before being taken up in an oil concentrate. The properties of the samples are shown in Table 3.

## TABLE 3

| | | Analysis of oil concentrate | | |
|---|---|---|---|---|
| Sample No | Time of continuous process h | BI eq Ca/eq SA | Ca-content %w | $V_k$ 100°C $mm^2/s$ |
| 1 | 1 | 13.6 | 13.0 | 18 |
| 2 | 4 | 13.7 | 12.5 | 17 |
| 3 | 7 | 15.5 | 13.6 | 20 |
| 4 | 7 | 16.1 | 16.2 | 42 |

## Claims

1. Process for the preparation of a basic alkaline earth metal salt of an organic carboxylic acid, which comprises
   (a) preparing a mixture of one equivalent of the organic carboxylic acid and at least one equivalent of an alkaline earth metal hydroxide and/or oxide in a hydrocarbon solvent and, optionally a promotor;
   (b) introducing carbon dioxide into the mixture in an amount of at least 0.5 equivalent carbon dioxide per equivalent alkaline earth metal; and
   (c) removing residual solids, if any, and the water layer, if any, obtained; characterised in that the carbonation reaction of step (b) is carried out at a temperature in the range of from 75 to 150°C.

2. Process according to claim 1, in which the organic carboxylic acid is alkylsalicylic acid having at least 10 carbon atoms in the alkyl group.

3. Process according to claim 1 or 2, in which the alkaline earth metal is calcium or magnesium.

4. Process according to any one of claims 1-3, in which the promotor is a $C_{1-6}$ alcohol and/or water.

5. Process according to any one of claims 1-4, in which the amount of alkaline earth metal hydroxide and/or oxide added in step (a) is from 10 to 25 equivalents per equivalent acid.

6. Process according to any one of claims 1-5, in which the introduction of carbon dioxide in step (b) is carried out at a temperature from 80 to 130°C, and a pressure between 1 and 5 bar (abs).

7. Process according to any one of claims 1-6, in which carbon dioxide is introduced in step (b) in an amount of 0.5 to 0.9 equivalent carbon dioxide per equivalent alkaline earth metal.

8. Process according to any one of claims 1-7, in which an ageing period from 0.25 to 20 hrs. is applied between steps (b) and (c).

9. Process according to any one of claims 1-8, in which steps (a) and (b) are carried out in two stages, whereby the stages comprise:-

(a1) preparing a mixture of one equivalent of the organic carboxylic acid and at least one equivalent of an alkaline earth metal hydroxide and/or oxide in a hydrocarbon solvent and optionally a $C_{1-6}$ alcohol and/or water;

(b1) introducing carbon dioxide into the mixture at a temperature from 75 to 150°C until at least 0.5 equivalent carbon dioxide per equivalent alkaline earth metal has been taken up;

(a2) adding at least one further equivalent of alkaline earth metal hydroxide and/or oxide to the reaction mixture, so that the total amount of alkaline earth metal hydroxide and/or oxide is at least 10 equivalent;

(b2) resuming the introduction of carbon dioxide to the resulting mixture at a temperature from 75 to 150°C.

**Patentansprüche**

1. Verfahren zur Herstellung eines basischen Erdalkalimetallsalzes einer organischen Carbonsäure, welches Verfahren umfaßt:

(a) Bereiten eines Gemisches aus einem Äquivalent der organischen Carbonsäure und wenigstens einem Äquivalent eines Erdalkalimetallhydroxids und/oder -oxids in einem Kohlenwasserstofflösungsmittel und gewünschtenfalls einem Promotor;

(b) Einführen von Kohlendioxid in das Gemisch in einer Menge von wenigstens 0,5 Äquivalenten Kohlendioxid je Äquivalent Erdalkalimetall; und

(c) Abtrennen gegebenenfalls vorhandener restlicher Feststoffe und der gegebenenfalls gebildeten Wasserphase; dadurch gekennzeichnet, daß die Carbonatisierungsreaktion von Stufe (b) bei einer Temperatur im Bereich von 75 bis 150°C ausgeführt wird.

2. Verfahren nach Anspruch 1, worin die organische Carbonsäure eine Alkylsalicylsäure mit einem Gehalt an wenigstens 10 Kohlenstoffatomen in der Alkylgruppe ist.

3. Verfahren nach Anspruch 1 oder 2, worin das Erdalkalimetall Calcium oder Magnesium ist.

4. Verfahren nach einem der Ansprüche 1 bis 3, worin der Promotor ein $C_{1-6}$ Alkohol und/oder Wasser ist.

5. Verfahren nach einem der Ansprüche 1 bis 4, worin die in Stufe (a) zugesetzte Menge an Erdalkalimetallhydroxid und/oder -oxid 10 bis 25 Äquivalente je Äquivalent Säure beträgt.

6. Verfahren nach einem der Ansprüche 1 bis 5, worin das Einführen von Kohlendioxid in Stufe (b) bei einer Temperatur von 80 bis 130°C und einem Druck zwischen 1 und 5 bar (absolut) vorgenommen wird.

7. Verfahren nach einem der Ansprüche 1 bis 6, worin das Kohlendioxid in der Stufe (b) in einer Menge von 0,5 bis 0,9 Äquivalenten Kohlendioxid je Äquivalent Erdalkalimetall eingeführt wird.

8. Verfahren nach einem der Ansprüche 1 bis 7, worin zwischen den Stufen (b) und (c) eine Alterungsperiode von 0,25 bis 20 Stunden eingehalten wird.

9. Verfahren nach einem der Ansprüche 1 bis 8, worin die Stufen (a) und (b) in zwei Stufen ausgeführt werden, wobei die Stufen umfassen:

(a1) Bereiten eines Gemisches aus einem Äquivalent der organischen Carbonsäure und wenigstens einem Äquivalent eines Erdalkalimetallhydroxids und/oder -oxids in einem Kohlenwasserstofflösungsmittel und gegebenenfalls einem $C_{1-6}$ Alkohol und/oder Wasser;

EP 0 267 658 B1

(b1) Einführen von Kohlendioxid in das Gemisch bei einer Temperatur von 75 bis 150°C, bis wenigstens 0,5 Äquivalente Kohlendioxid je Äquivalent Erdalkalimetall aufgenommen worden sind;
(a2) Zusetzen wenigstens eines weiteren Äquivalents Erdalkalimetallhydroxid und/oder -oxid zum Reaktionsgemisch, sodaß die Gesamtmenge an Erdalkalimetallhydroxid und/oder -oxid wenigstens 10 Äquivalente beträgt;
(b2) Wiederaufnehmen des Einführens von Kohlendioxid in das gebildete Gemisch bei einer Temperatur von 75 bis 150°C.

**Revendications**

1. Procédé pour la préparation d'un sel basique de métal alcalino-terreux d'un acide organique carboxylique, qui consiste à :
(a) préparer un mélange d'un équivalent de l'acide carboxylique et d'au moins un équivalent d'un hydroxyde et/ou d'un hydroxyde de métal alcalino-terreux dans un solvant à base d'hydrocarbone, et, facultativement un promoteur;
(b) introduire le dioxyde de carbone dans le mélange en une quantité d'eau d'au moins 0,5 équivalent de dioxyde de carbone, par équivalent de métal alcalino terreux; et
(c) éliminer les solides résiduels, s'il y en a, et l'eau, si elle existe, obtenus ; caractérisé en ce que la réaction de carbonation de l'étape (b) est mise en oeuvre à une température dans la gamme de 75 à 150°C.

2. Procédé selon la revendication 1, dans lequel l'acide organique carboxylique est un acide alkylsalicyclique présentant au moins 10 atomes de carbone dans le groupe alkyle.

3. Procédé selon la revendication 1 ou 2, dans lequel le métal alcalino-terreux est le calcium ou le magnésium.

4. Procédé selon l'une quelconque des revendications 1 à 3, dans lequel le promoteur est un alcool en $C_1$ à $C_6$ et/ou de l'eau.

5. Procédé selon l'une quelconque des revendications 1 à 4, dans lequel la quantité d'hydroxyde et/ou d'oxyde de métal alcalino-terreux ajoutée à l'étape (a) est de 10 à 25 équivalents par équivalent d'acide.

6. Procédé selon l'une quelconque des revendications 1 à 5, dans lequel l'introduction de dioxyde de carbone à l'étape (b) est mise en oeuvre à une température de 80 à 130°C, et sous une pression entre 1 et 5 bars (abs).

7. Procédé selon l'une quelconque des revendications 1 à 6, dans lequel le dioxyde de carbone est introduit à l'étape (b) en une quantité de 0,5 à 0,9 équivalent de dioxyde de carbone par équivalent de métal alcalinoterreux.

8. Procédé selon l'une quelconque des revendications 1 à 7, dans lequel une période de vieillissement de 0,25 à 20 heures est appliquée entre les étapes (b) et (c).

9. Procédé selon l'une quelconque des revendications 1 à 8, dans lequel les étapes (a) et (b) sont mises en oeuvre en deux stades, ces étapes consistant à :
(a1) préparer un mélange d'un équivalent d'acide organique carboxylique et d'au moins un équivalent d'un hydroxyde et/ou d'un oxyde de métal alcalino-terreux dans un solvant à base d'hydrocarbone, et, facultativement dans un alcool en $C_1$ à $C_6$ et/ou dans l'eau;
(b1) introduire le dioxyde de carbone dans le mélange à une température de 75 à 150°C jusqu'à ce qu'au moins 0,5 équivalent de dioxyde de carbone par équivalent de métal alcalino-terreux ait été capté;
(a2) ajouter au moins un autre équivalent d'hydroxyde et/ou d'oxyde de métal alcalino-terreux au mélange réactionnel de façon que la quantité totale d'hydroxyde et/ou d'oxyde de métal alcalino-terreux soit d'au moins 10 équivalents;
(b2) reprendre l'introduction du dioxyde de carbone dans le mélange résultant à une température de 75 à 150°C.